# DEMANDE DE BREVET EUROPEEN

(11) **EP 2 181 965 A1**
(43) Date de publication de la demande: **05.05.2010**
(21) Numéro de dépôt: 09352006.2
(22) Date de dépôt: 30.10.2009
(51) Int. Cl.: C02F 1/44

(54) **Installation et procédé de traitement d'eau pour dialyse**

(30) Priorité: 30.10.2008 FR 0806032
(71) Demandeur: Commercialisation-Recherche S-Realisation, 31450 Pompertuzat (FR)
(72) Inventeur: Grangé, Serge, 31320 Castanet-Tolosan (FR)
(74) Mandataire: Hartmann, Jean-Luc

(57) **Abrégé**

Un procédé de traitement d'eau selon l'invention pour la production d'eau pour hémodialyse est prévu pour traiter une eau potable déchlorée et filtrée.

L'eau à traiter est mise sous haute pression, au-delà de 2,5 MPa, puis est traitée par osmose inverse dans un premier étage (26) comportant une membrane destinée à être utilisée pour des eaux contenant au moins 10 g de chlorure de sodium (NaCl) par litre.

En sortie du premier étage (26), l'eau alimente un second étage (28) dans lequel elle est traitée par osmose inverse, le premier étage (26).

L'invention concerne également un dispositif pour la mise en oeuvre de ce procédé.

## Description

La présente invention concerne un système de traitement et de distribution d'eau pour dialyse.

Pour purifier le sang de personnes souffrant d'insuffisance rénale, il est connu de réaliser une dialyse extra-corporelle du sang. Le sang du patient passe alors dans un circuit de circulation extracorporelle. II est traité par un appareil appelé générateur et qui permet de retirer du sang les déchets produits par l'alimentation du patient, d'équilibrer le bilan électrolytique sanguin du patient ainsi que d'éliminer le surplus d'eau accumulé dans le corps du patient.

Plusieurs techniques de dialyse existent. Dans le domaine du traitement de l'insuffisance rénale, les principales techniques utilisées sont l'hémodialyse, l'hémodiafiltration et l'hémofiltration.

Pour réaliser une telle dialyse, de manière connue de l'homme du métier, il est nécessaire d'obtenir une eau très pure. La présente invention concerne la production d'une eau utilisée pour réaliser une dialyse.

Dans un domaine technique différent de celui de la présente invention, le document WO-98/31637 révèle un procédé de prétraitement non chimique destiné à produire de l'eau ultrapure, dont les étapes essentielles consistent à soumettre un charge d'alimentation aqueuse à un champ d'énergie électromagnétique en vue de provoquer une polarisation et une agglomération de matières particulaires en suspension, et procéder ensuite à une filtration sur lit multicouche pour retirer ces matières particulaires.

Généralement, pour produire de l'eau pour une dialyse, plusieurs techniques sont associées. Ainsi, la production d'eau comporte plusieurs étapes telles généralement : filtration, adoucissement, passage sur des charbons actifs, filtration et osmose inverse. Les caractéristiques de ces différentes étapes dépendent notamment de l'eau brute qui est utilisée en entrée du dispositif pour réaliser l'eau pure destinée au générateur de dialyse. II convient également de tenir compte des caractéristiques spécifiques du générateur.

Ainsi, les procédés de l'art antérieur prévoient une ou plusieurs étapes pour éliminer les particules en suspension par filtration ainsi que les sels à l'aide de résines d'échange de cations. II convient de prévoir des périodes de régénération pour l'échange d'ions. L'étape utilisant des charbons actifs est une étape d'adsorption qui est réalisée pour éliminer le chlore contenu dans l'eau à traiter. Une étape de filtration est ensuite réalisée pour retenir les fines particules libérées par le charbon actif utilisé. En général, l'étape finale du traitement est réalisée par osmose inverse pour l'hémodialyse et par double osmose conventionnelle pour l'hémofiltration et l'hémodiafiltration afin d'éliminer toutes les molécules résiduelles de l'eau traitée.

Dans les procédés actuels, le chainage complet du procédé ne peut être défini car il dépend le plus souvent de la qualité d'eau d'alimentation. En outre, les techniques d'échange d'ions et d'adoucissement utilisent des consommables, tels des sels de régénération et des résines échangeuses d'ions, ce qui engendre des coûts de fonctionnement et une exploitation complexe. En outre, avec les procédés de l'art antérieur, il y a un risque de pollution bactériologique car le matériel utilisé est le plus souvent un piège à germes.

La présente invention a alors pour but de fournir un procédé et un dispositif permettant de traiter l'eau afin d'obtenir une eau pure indépendamment de l'analyse physicochimique et biologique de l'eau potable d'entrée. De préférence, le coût pour la mise en oeuvre de ce procédé sera inférieur aux coûts constatés avec les procédés de l'art antérieur. De même, le procédé selon l'invention engendrera avantageusement une maintenance réduite.

À cet effet, la présente invention propose un procédé de traitement d'eau pour la production d'eau pour dialyse à partir d'eau potable déchlorée et filtrée.

Selon la présente invention, ce procédé prévoit que l'eau à traiter est mise sous haute pression, au-delà de 2,5 MPa (soit au-delà de 25 bars), puis est traitée par osmose inverse dans un premier étage, et que l'eau en sortie du premier étage alimente un second étage dans lequel elle est traitée par osmose inverse, le premier étage comportant au moins une membrane destinée à être utilisée pour des eaux d'une salinité supérieure à 25 g par litre.

Ce procédé prévoit donc uniquement une première osmose inverse avec une membrane à fort pouvoir de rétention des sels, telle une membrane utilisée habituellement pour un traitement de désalinisation afin d'obtenir une eau douce, et une deuxième osmose inverse pour une seconde élimination des sels. II permet ainsi d'obtenir une eau pure en sortie sans qu'il ne soit nécessaire de prévoir une opération d'adoucissement et/ou d'échange d'ions.

Pour obtenir un pression suffisante au niveau du second étage du procédé sans avoir besoin de rajouter une pompe ou similaire entre les deux étages, l'eau est avantageusement surpressée en amont du premier étage à une pression supérieure à 4 MPa. On utilisera ici de préférence une pompe volumétrique qui permet de donner une pression constante.

La membrane du premier étage est de préférence une membrane adaptée pour fournir une eau douce par désalinisation. Une telle membrane est adaptée à une salinité élevée et travaille généralement à haute pression. L'utilisation d'une telle membrane pour une eau potable est tout à fait inhabituelle pour l'homme du métier.

La membrane du premier étage est par exemple aussi **caractérisée en ce qu**'elle est adaptée pour des eaux ayant une conductivité supérieure à 40 mS/cm. On retrouve ici la plupart des membranes adaptées pour fournir une eau douce par désalinisation.

On peut éventuellement prévoir d'inclure dans un procédé de traitement d'eau décrit ci-dessus l'étape permettant d'obtenir une eau déchlorée. Cette déchloration est par exemple réalisée à l'aide de charbons actifs. Une filtration devra généralement être réalisée après le passage sur les charbons actifs pour éliminer de fines particules qui sont mises en suspension dans l'eau au cours du passage sur les charbons actifs.

La présente invention concerne également un dispositif pour le traitement d'eau pour la production d'eau pour dialyse à partir d'eau potable déchlorée et filtrée, comportant deux étages d'osmose inverse, **caractérisé en ce qu**'il comporte en amont des deux étages des moyens de mise sous haute pression permettant de mettre l'eau à traiter à une pression supérieure à 2,5 MPa, en ce que les deux étages sont montés en série, et en ce que le premier étage est équipé d'au moins une membrane destinée à être utilisée pour des eaux d'une salinité supérieure à 25 g par litre.

Un tel dispositif permet de réaliser un procédé de traitement d'eau tel que décrit plus haut.

Dans un tel dispositif, les moyens de mise sous haute pression permettent de préférence de mettre l'eau à traiter à une pression supérieure à 4 MPa. Ces moyens sont avantageusement une pompe volumétrique qui permet de délivrer une pression constante.

De même que pour le procédé selon l'invention, la membrane du premier étage est par exemple une membrane adaptée pour fournir une eau douce par désalinisation et/ou une membrane adaptée pour des eaux ayant une conductivité supérieure à 40 mS/cm.

Dans un procédé tel que décrit plus haut, ou dans le dispositif ici, on peut prévoir avantageusement que chaque membrane du premier étage est destinée à être utilisée pour des eaux d'une salinité supérieure à 30 g par litre et/ou que chaque membrane du second étage est une membrane de biosmose destinée à être utilisée avec des eaux avec une faible salinité, inférieure à 2,5 g par litre. On a alors un premier étage avec des membranes destinées au désalement d'eau de mer et un second étage avec des membranes "classiques" utilisées en biosmose.

Dans un tel dispositif, pour limiter les frais de fabrication, les moyens de mise sous pression de l'eau à traiter en amont du premier étage sont les seuls moyens de mise sous pression de l'eau à traiter utilisés dans le dispositif.

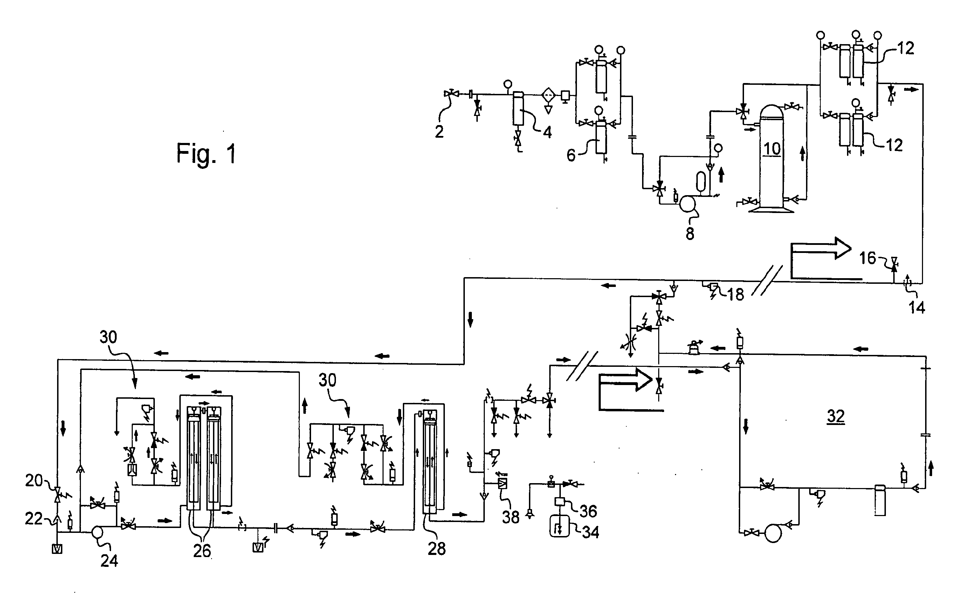

Des détails et avantages de la présente invention ressortiront mieux de la description qui suit, faite en référence au dessin schématique annexé sur lequel :
L'unique figure représente un exemple de réalisation d'un dispositif de traitement d'eau selon la présente invention.
La figure jointe représente un dispositif de traitement selon l'invention ainsi qu'un dispositif de filtration et de déchloration qu'il convient le plus souvent de prévoir en amont d'un dispositif selon l'invention.
Pour retirer le chlore d'une eau, il est connu de l'homme du métier de faire passer cette eau sur des charbons actifs. Il convient alors de filtrer l'eau ainsi déchlorée afin d'éliminer les particules provenant du traitement mettant en oeuvre les charbons actifs.
Sur l'unique figure, on reconnaît une vanne d'entrée 2 pour commander l'alimentation en eau potable de l'ensemble du dispositif représenté. Cette eau est tout d'abord filtrée dans un filtre 4 filtrant à 100 microns (100 µm). Après passage dans un disconnecteur et un détendeur afin de protéger l'installation d'eau et de maîtriser la pression de celle-ci, l'eau pré-filtrée dans le filtre 4 passe ensuite dans deux filtres 6 filtrant chacun à 10 microns et montés en parallèle.

L'eau est ensuite surpressée par une première pompe 8 qui délivre une pression de l'ordre de quelques bars pour envoyer l'eau filtrée dans un corps 10 contenant des charbons actifs. En sortie de ce corps 10, l'eau est déchlorée. Elle contient toutefois de fines particules provenant des charbons actifs se trouvant dans le corps 10. Une filtration est alors réalisée en aval du corps 10 par un ensemble de quatre filtres 12.

Une vérification de la qualité de l'eau ainsi traitée est réalisée d'une part par une sonde de résistivité 14 et d'autre part par un chloromètre 16.

Un tel dispositif pour retirer le chlore d'eau potable est connu de l'homme du métier. Tout autre dispositif connu de l'art antérieur peut être utilisé ici pour fournir une eau déchlorée et filtrée au dispositif de fabrication d'eau pure décrit ci-après.

En entrée du dispositif de traitement d'eau, on trouve de manière classique un débitmètre 18, une électrovanne 20 et une soupape anti-retour 22. On ne décrira pas ici dans le détail les divers éléments tels des vannes, des manomètres, des éléments de sécurité et des éléments de contrôle qui sont des éléments habituels dans tout dispositif hydraulique et qui sont connus de l'homme du métier. En outre, tous ces éléments peuvent être placés différemment par rapport à la forme de réalisation représentée au dessin.

Ainsi, l'eau est tout d'abord surpressée par une pompe haute pression 24. Cette pompe est de préférence une pompe volumétrique. Elle est par exemple montée sur silent-blocs. En sortie de cette pompe, l'eau se trouve à une pression supérieure à 25 bars et de préférence supérieure à 40 bars (soit supérieure à 4 MPa), par exemple à 45 bars (4,5 MPa). Pour bien gaver cette pompe, il convient de veiller à ce que l'eau en entrée de cette pompe haute pression 24 soit en pression, de l'ordre de quelques bars.

Cette eau sous haute pression est alors traitée par un procédé d'osmose inverse dans un premier étage. Pour ce faire, dans l'exemple de réalisation montré sur l'unique figure, l'eau est envoyée dans deux premiers corps de pression 26 montés en série, les sorties de ces corps de pression étant toutefois reliées en parallèle. Ces premiers corps de pression 26 sont tout d'abord conçus pour travailler aux pressions élevées délivrées par la pompe haute pression 24. Ces premiers corps de pression 26 contiennent en outre chacun une membrane à fort pouvoir de coupure de salinité. Ce pouvoir est largement supérieur au pouvoir de coupure de salinité rencontré sur des membranes de réacteurs d'osmose inverse utilisés pour de l'eau potable.

Dans une forme de réalisation préférée, et non limitative, les membranes utilisées au niveau du premier étage sont des membranes pour osmose inverse utilisés pour fournir une eau douce par désalinisation, par exemple par désalinisation d'eau de mer. À titre d'exemple, il peut s'agir de membranes commercialisées par la Société "Toray" et faisant partie de la gamme TM800 de ce fabricant. Il s'agit d'une membrane en matière composite entièrement en polyamide aromatique réticulé (en anglais : Cross Linked Fully Aromatic Polyamide Composite). Une telle membrane accepte des pressions allant jusqu'à 5,52 MPa (soit environ 55 bars). Elle accepte également une eau à forte salinité allant jusqu'à 32 g/l de NaCl (chlorure de sodium).

À la sortie de ce premier étage, l'eau est encore à une pression suffisante (de l'ordre de 10 à 15 bars) pour pouvoir alimenter un second étage comportant un second corps de pression 28 contenant une membrane pour osmose inverse. Ce second corps de pression 28 contient une membrane "classique", telle celles utilisées habituellement pour réaliser de l'eau pure dans le domaine de la dialyse. On retrouve alors une membrane avec une coupure de salinité de l'ordre 500 à 1 500 mg/l. Une membrane de ce type se trouve classiquement dans un dispositif de traitement d'eau pour la préparation d'eau pure pour une hémodialyse. À titre d'exemple non limitatif, il s'agit ici par exemple d'une membrane commercialisée par la Société "Toray" sous l'une des références SU-710, SU-710L, SUL-G10. Il peut aussi s'agir par exemple d'une membrane commercialisée par cette même Société et faisant partie de la gamme TM700 ou TMG ou TMH.

On réalise ainsi deux étages d'osmose inverse qui permettent d'obtenir une eau pure. Pour chaque étage d'osmose, un circuit de rejet 30 est prévu pour les eaux de rejet, ou concentrat. On remarque sur l'unique figure que le circuit de rejet 30 du second étage est raccordé également en amont de la pompe haute pression 24 permettant ainsi d'assurer un recyclage du rejet du second étage.

À la sortie du second étage, comprenant le second corps de pression 28 et sa membrane associée, l'eau est envoyée vers un circuit de distribution 32. Ce circuit n'est pas décrit plus en détails ici. Il correspond à un circuit de distribution classique et ne fait pas partie de la présente invention. Il s'agit par exemple d'un circuit bouclé sans volume mort avec une gestion automatique de retour de boucle. La vitesse de passage est par exemple contrôlable, de 1 à 1,5 m/s.

À la fin de la ligne de traitement d'eau, on prévoit également des moyens de désinfection pour garantir la qualité du produit délivré. On trouve ainsi une cuve 34 contenant un produit désinfectant et associée à une pompe doseuse 36. Ces moyens de désinfection peuvent venir se brancher sur le circuit de traitement d'eau au niveau d'une poignée 38, en aval du second étage.

Le dispositif de traitement d'eau décrit ci-dessus permet de fournir une gamme de dialyse modulable en fonction du nombre de postes de dialyse souhaités.

Le traitement ne dépend plus de la qualité de l'eau en entrée mais uniquement du cahier des charges du client concernant l'eau à la sortie du dispositif de traitement. De ce fait, la fabrication de ce dispositif de traitement peut être rationnalisé et il est envisageable de prévoir une fabrication en série.

Grâce notamment à la présence d'une seule pompe et à l'absence d'un adoucisseur et d'un échangeur d'ions, le prix de revient d'un tel dispositif de traitement est inférieur au prix de revient d'un dispositif comparable de l'art antérieur. Globalement, on réalise ici une double osmose pour le prix d'un dispositif de l'art antérieur ne réalisant qu'un seul étage d'osmose.

En outre, le coût de fonctionnement et la maintenance de ce dispositif sont réduits par rapport aux dispositifs comparables de l'art antérieur. En effet, il n'y a pas ici de traitement d'eau préalable à la réalisation de l'étape d'osmose. De ce fait, il est inutile de prévoir des moyens pour adoucir l'eau qui demandent une maintenance régulière. En effet, le procédé décrit ci-dessus permet d'éviter un prétraitement de l'eau et en particulier un prétraitement par résine de régénération. L'osmose inverse réalisée ici permet en effet la rétention de pratiquement tous les composés présents en solution dans l'eau à traiter.

Des tests réalisés sur un pilote ont montré que le procédé décrit ci-dessus en référence au dispositif présenté sur la figure jointe, permet d'obtenir une eau conforme à la pharmacopée européenne pour la production d'eau pour hémodialyse et hémodiafiltration. Une triple qualification, opérationnelle, biologique (bactéries et endotoxines) et physico-chimique, a été réalisée et a validé le procédé de traitement selon la présente invention.

La présente invention permet également de réaliser un dispositif de traitement d'eau bien plus compact que les dispositifs de l'art antérieur. Ceci est ici intéressant car le volume des dispositifs de traitement est important et le gain de place réalisé par la présente invention est sensible.

La présente invention ne se limite pas au procédé et au dispositif décrits ci-dessus à titre d'exemples non limitatifs. Elle concerne également toutes les variantes de réalisation à la portée de l'homme du métier dans le cadre des revendications ci-après.

Ainsi, les valeurs numériques indiquées dans la description sont donnés à titre purement illustratifs.

Dans le premier étage d'un dispositif de traitement selon l'invention, il est possible de ne prévoir qu'une seule membrane d'osmose inverse. Il est important ici que cet étage présente au moins une membrane ayant un pouvoir de rétention important et un fort pouvoir de coupure de salinité. Il faut également que cette membrane supporte les pressions élevées fournies par la pompe haute pression. Lorsque plusieurs membranes sont utilisées, divers montages de celles-ci -et de leurs corps de pression- peuvent être envisagés.

Pour le second étage, seule une membrane a été prévue dans la description faite plus haut. Toutefois, il est évidemment possible d'avoir également plusieurs membranes. Tout ceci dépend bien entendu de l'importance de l'installation.

## Revendications

1. Procédé de traitement d'eau pour la production d'eau pour dialyse à partir d'eau potable déchlorée et filtrée, **caractérisé en ce que** l'eau à traiter est mise sous haute pression, au-delà de 2,5 MPa, puis est traitée par osmose inverse dans un premier étage (26), et **en ce que** l'eau en sortie du premier étage (26) alimente un second étage (28) dans lequel elle est traitée par osmose inverse, le premier étage (26) comportant au moins une membrane destinée à être utilisée pour des eaux d'une salinité supérieure à 25 g par litre.

2. Procédé de traitement selon la revendication 1, **caractérisé en ce que** l'eau est pompée en amont du premier étage (26) à une pression supérieure à 4 MPa.

3. Procédé de traitement selon l'une des revendications 1 ou 2, **caractérisé en ce que** la membrane du premier étage (26) est une membrane adaptée pour fournir une eau douce par désalinisation.

4. Procédé de traitement selon l'une des revendications 1 à 3, **caractérisé en ce que** la membrane du premier étage (26) est adaptée pour des eaux ayant une conductivité supérieure à 40 mS/cm.

5. Procédé de traitement d'eau selon l'une des revendications 1 à 4, **caractérisé en ce que** l'eau est au préalable déchlorée à l'aide de charbons actifs.

6. Procédé selon l'une des revendications 1 à 5, **caractérisé en ce que** chaque membrane du premier étage est destinée à être utilisée pour des eaux d'une salinité supérieure à 30 g par litre.

7. Procédé selon l'une des revendications 1 à 6, **caractérisé en ce que** chaque membrane du second étage est une membrane de biosmose destinée à être utilisée avec des eaux avec une faible salinité, inférieure à 2,5 g par litre.

8. Dispositif pour le traitement d'eau pour la production d'eau pour dialyse à partir d'eau potable déchlorée et filtrée, comportant deux étages (26, 28) d'osmose inverse, **caractérisé en ce qu'il** comporte en amont des deux étages (26, 28) des moyens de mise sous haute pression (24 ) permettant de mettre l'eau à traiter à une pression supérieure à 2,5 MPa, **en ce que** les deux étages (26, 28) sont montés en série, et **en ce que** le premier étage (26) est équipé d'au moins une membrane destinée à être utilisée pour des eaux d'une salinité supérieure à 25 g par litre.

9. Dispositif pour le traitement d'eau selon la revendication 8, **caractérisé en ce que** les moyens de mise sous haute pression (24) permettent de mettre l'eau à traiter à une pression supérieure à 4 MPa.

10. Dispositif pour le traitement d'eau selon l'une des revendications 8 ou 9, **caractérisé en ce qu'**au moins une membrane du premier étage (26) est une membrane adaptée pour fournir une eau douce par désalinisation.

11. Dispositif pour le traitement d'eau selon l'une des revendications 8 à 10, **caractérisé en ce qu'**au moins une membrane du premier étage (26) est adaptée pour des eaux ayant une conductivité supérieure à 40 mS/cm.

12. Dispositif pour le traitement d'eau selon l'une des revendications 8 à 11, **caractérisé en ce que** les moyens de mise sous pression (24) de l'eau à traiter en amont du premier étage sont les seuls moyens de mise sous pression de l'eau à traiter utilisés dans le dispositif.

13. Dispositif selon l'une des revendications 8 à 12, **caractérisé en ce que** chaque membrane du premier étage est destiné à être utilisée pour des eaux d'une salinité supérieure à 30 g par litre.

14. Dispositif selon l'une des revendications 8 à 13, **caractérisé en ce que** chaque membrane du second étage est une membrane de biosmose destinée à être utilisée avec des eaux avec une faible salinité, inférieure à 2,5 g par litre.
